# EUROPEAN PATENT APPLICATION

(11) **EP 1 206 922 A1**
(43) Date of publication of application: **22.05.2002**
(21) Application number: 01126943.8
(22) Date of filing: 13.11.2001
(51) Int. Cl.: A61B 5/00, A61B 5/0205

(54) **Device for measuring body cavity temperature**

(30) Priority: 16.11.2000 IL 13972100
(71) Applicant: Alcor Medical Instruments Ltd., 91353 Jerusalem (IL)
(72) Inventor: Abramovitch, Aaron, Givat Ze'ev, Jerusalem (IL); Lozinski, Yuli, Gilo, Jerusalem 93846 (IL)
(74) Representative: Frauenknecht, Alois J.

(57) **Abstract**

A device for sensing the temperature inside a body cavity having inside walls, includes a tube having, at its proximal end, an electrical wire connector and an air admittance and release mechanism, and, at its distal end, an inflatable annular cuff operationally connected to the mechanism; temperature-sensing means attached to, or integral with, the cuff and electically connected to the wire connector; the sensing means being located at the region of the largest diameter of the annular cuff when inflated, the inflation of the cuff temporarily affixing the cuff to the inside walls of the body cavity while assuring at least indirect positive contact between the sensing means and the body cavity.

## Description

### Field of the Invention

The present invention relates to a device for sensing temperature inside a body cavity.

### Background of the Invention

The provision of an accurate measurement of the temperature of a body cavity during surgery, general anesthesia, and other critical care circumstances, is an important aspect of the clinical monitoring, evaluation and management of a patient's condition. The provision of blood transfusions and/or too warm intravenous fluids may lead to a significant elevation of the patient's body temperature, thus increasing the body's oxygen consumption, which may cause hypoxia of the brain and other vital organs. In addition, the provision of too cold intravenous solutions may lead to hypothermia, which leads to a decrease of oxygen in the body. Complications such as malignant hyperthermia resulting from general anesthesia, may lead to the loss of life if not appropriately recognized.

Currently, a number of temperature sensors for body cavity temperature measurement are known and in use. These devices include the tympanic membrane thermometer, anal thermometer, distal esophagus temperature probe, and pulmonary artery and nasopharynx temperature probes. The tympanic membrane thermometer, rectal and nasopharynx temperature probes must be carefully located and may be easily disconnected from the site during surgery; in addition, they cause discomfort to the patient after surgery. The placement of a pulmonary artery temperature probe is an invasive procedure, requiring the performance of pulmonary artery catheterization, The esophageal temperature probe must also be carefully placed, and it may be influenced by cooling inspired air in the adjacent trachea.

Furthermore, during surgery, the anesthesiologist is busy monitoring the patient's vital body functions; every additional procedure, such as placing and caring for additional probes, may disturb his more important tasks.

U.S. Patent No. 5,622,182 relates to the measurement of body temperature from the lumen of the trachea and/or from the endotracheal tube inserted into the trachea. The system shown is complex, including a body cavity temperature-sensing device and a respiratory gas-sensing device, and is based on a correlation between temperature samplings and respiration phases, processing the temperature values of expired gas. This system is cumbersome and expensive, and it is questionable whether inspired gas temperature is an accurate expression of the body cavity temperature.

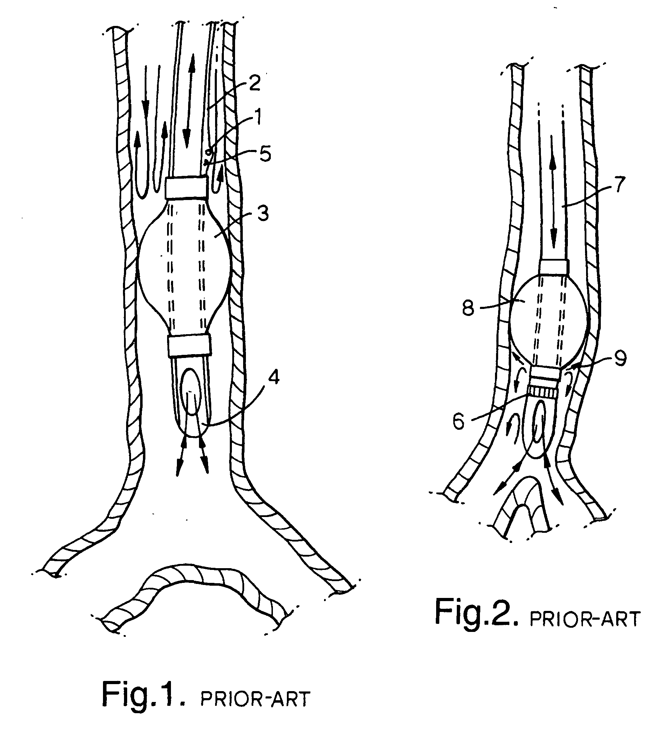

U.S. Patent No. 4,263,921 relates to a temperature-sensing method and endotracheal tube appliance. As can be seen in Fig. 1 of the present application, illustrating this prior art device, the temperature sensor or thermistor 1 is mounted on the tube 2 proximate the end of the inflatable cuff 3 and remote from the free distal end portion 4. The portion 5 of the outer face of the tube, which extends across the thermistor 1, slightly bulges from the adjacent portion of the outer face, with the aim of providing better contact with the trachea. The disadvantages of this device are as follows: As shown by the arrowed lines, the placement of the temperature sensor 1 would be influenced by inspired gases inside the lumen of the endotracheal tube 2 and by air from the nasopharynx circulating up to the proximal end of the inflated cuff 3, both in the vicinity of thermistor 1, inside and outside the trachea.

German Publication No. 19543072 A1 describes an endotracheal tube for continuous body cavity temperature measurement. As shown in prior art Fig. 2 of the present application, the temperature sensor 6 is located on the distal tip of the tube 7, beneath the inflatable cuff 8. It is not specified what kind of sensor is used and how it is integrated, if at all, into the walls of the tube. As can be seen, the sensor 6 is located at a substantial distance from the tracheal mucosa and is in close proximity to the site from which expired and inspired gases enter into the trachea and thence into the tube. The additional cooling influence provides local gas turbulence at 9, thereby preventing the accurate measurement of body cavity temperature.

### Disclosure of the Invention

It is a broad object of the present invention to provide a device for accurately sensing the temperature inside a body cavity.

The invention therefore provides a device for sensing the temperature inside a body cavity having inside walls, comprising a tube having, at its proximal end, an electrical wire connector and an air admittance and release mechanism, and, at its distal end, an inflatable annular cuff operationally connected to said mechanism; temperature-sensing means attached to, or integral with, said cuff and electrically connected to said wire connector; said sensing means being located at the region of the largest diameter of said annular cuff when inflated, the inflation of said cuff temporarily affixing the cuff to the inside walls of the body cavity while assuring at least indirect positive contact between said sensing means and said body cavity.

### Brief Description of the Drawings

The invention will now be described in connection with certain preferred embodiments with reference to the following illustrative figures so that it may be more fully understood.

With specific reference now to the figures in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Figs. 1 and 2 illustrate prior art body cavity temperature-sensing devices;
Fig. 3 illustrates the body cavity temperature-sensing device of the present invention,
   and
Fig. 4 illustrates another embodiment of the device of the invention.

### Detailed Description

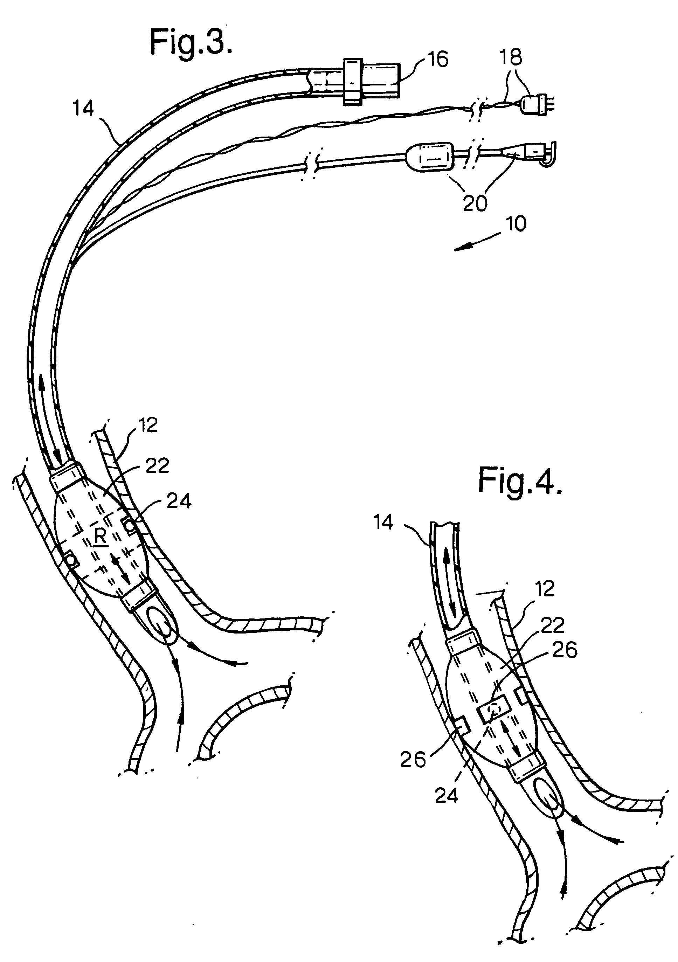

There is illustrated in Fig. 3 a device 10 for accurately measuring the temperature of a body cavity 12, e.g., a tracheal mucosa. The device 10 includes *per se* known endotracheal tube 14, having, at its proximal end, a tube connector 16, electrical wires and connector 18, and air admittance and release mechanism 20 for controlled inflation and deflation of an annular cuff 22 located at the distal end of the tube 14. In contradistinction to the prior art devices, the cuff according to the present invention is fitted with at least one temperature-sensing means 24, e.g., a thermistor. Sensing means 24 may be attached to the outer surface of cuff 22 or at least partly embedded therein. Advantageously, sensing means 24 is affixed on the outer surface of cuff 22 at the region *R* of the largest diameter of the inflated cuff, e.g., at its mid-portion.

Temperature-sensing means 24 may consist of a plurality of electrically interconnected individual sensors, for example, two sensors located at diametrically opposite locations around the cuff as shown in Fig. 3, or may be made in the form of one or more elongated strips 26 (Fig. 4). Alternatively, the temperature-sensing means 24 may be composed of one or more individual sensors covered or contacted by a strip 26 made of highly conductive thermal material, e.g., a metallic film, extending around the mid-portion of cuff 22. Obviously, cuff 22 may be made integrally with one or more sensors, and/or with a combination of one or more sensors and a thermally conductive strip 26 as described above.

Electrical wires and connector 18 transmit temperature-related signals to suitable, known measuring, monitoring and processing means.

As will be understood, the air-inflatable cuff 22 has a dual function: it temporarily affixes the cuff-borne sensors to the walls of the body cavity, the temperature of which is to be measured and monitored, assuring continuous, positive contact between the sensors and the cavity walls during the entire period of measurement, as well as providing a thermal insulator, eliminating unwanted thermal influences on the sensors and on the measured temperature.

It will be evident to those skilled in the art that the invention is not limited to the details of the foregoing illustrated embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A device for sensing the temperature inside a body cavity having inside walls, comprising:
a tube having, at its proximal end, an electrical wire connector and an air admittance and release mechanism, and, at its distal end, an inflatable annular cuff operationally connected to said mechanism;
temperature-sensing means attached to, or integral with, said cuff and electrically connected to said wire connector;
said sensing means being located at the region of the largest diameter of said annular cuff when inflated, the inflation of said cuff temporarily affixing the cuff to the inside walls of the body cavity while assuring at least indirect positive contact between said sensing means and said body cavity.

2. The device as claimed in claim 1, wherein said temperature-sensing means is affixed to an outside surface of said inflatable cuff.

3. The device as claimed in claim 1, wherein said temperature-sensing means is at least partly embedded in an outside surface of said inflatable cuff.

4. The device as claimed in claim 1, wherein said temperature-sensing means is constituted by one or more thermistors.

5. The device as claimed in claim 1, wherein said temperature-sensing means is in the form of one or more strips affixed to the outside surface of said cuff.

6. The device as claimed in claim 1, further comprising one or more strips of thermally conductive material attached to said cuff in thermal contact with said temperature-sensing means.

7. The device as claimed in claim 1, wherein several temperature-sensing means are located around die perimeter of said cuff in spaced-apart relationship.
